(19) Europäisches Patentamt
European Patent Office
Office européen des brevets

(11) Publication number: 0 117 501
B1

(12) # EUROPEAN PATENT SPECIFICATION

(45) Date of publication of patent specification: 06.04.88

(51) Int. Cl.[4]: C 07 C 125/073, C 07 D 209/48

(21) Application number: 84101745.2

(22) Date of filing: 20.02.84

(54) A process for the preparation of 1,5-diprotected-1,5,10-triazadecane, intermediates thereof and their preparation.

(30) Priority: 24.02.83 JP 28499/83

(43) Date of publication of application:
05.09.84 Bulletin 84/36

(45) Publication of the grant of the patent:
06.04.88 Bulletin 88/14

(84) Designated Contracting States:
AT BE CH DE FR GB IT LI LU NL SE

(56) References cited:
DE-A-2 102 559
GB-A- 744 587
US-A-3 341 568

HOUBEN-WEYL "Methoden der organischen Chemie", 4th edition, vol. XV/1: "Synthese von Peptiden Teil I", 1974, GEORG THIEME VERLAG, Stuttgart, pages 250-263

THE JOURNAL OF ANTIBIOTICS, vol. 34, no. 12, 1981, S. KONDO et al. "The total synthesis of spergualin, an antitumor antibiotic", pages 1625-1627

(73) Proprietor: MICROBIAL CHEMISTRY RESEARCH FOUNDATION
14-23, Kamiosaki 3 Chome Shinagawa-ku
Tokyo 141 (JP)

(72) Inventor: Umezawa, Hamao, Prof.
4-23, Toyotama-kita
Nerima-ku Tokyo (JP)
Inventor: Nishizawa, Rinzo
3-18-14, Sugamo
Toshima-ku Tokyo (JP)
Inventor: Takei, Yukio
7-3-5, Namiki
Abiko-city Chiba Prefecture (JP)
Inventor: Yoshida, Masao
A-611, 2-5-18, Daimon-cho
Higashikurume-city Tokyo (JP)
Inventor: Suzuki, Masao
3-17-1-203, Shimo
Kita-ku Tokyo (JP)
Inventor: Tomiyoshi, Tsugio
3-17-10, Shimo
Kita-ku Tokyo (JP)
Inventor: Nakamura, Teruya
831-6, Nomura-cho
Kusatsu-city Shiga Prefecture (JP)

EP 0 117 501 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(74) Representative: **Meyer-Dulheuer, Karl-Hermann, Dr. et al**
**HOECHST Aktiengesellschaft Zentrale**
**Patentabteilung Postfach 80 03 20**
**D-6230 Frankfurt/Main 80 (DE)**

**Description**

The present invention relates or intermediates for the preparation of 1,5-diprotected-1,5,10-triazadecanes and to a process for preparing these intermediates.

The present invention relates to 10-phthalyl-1,5-diprotected-1,5,10-triazadecanes of the general formula II and to a process for preparing the same, which comprises reacting a compound of the formula III

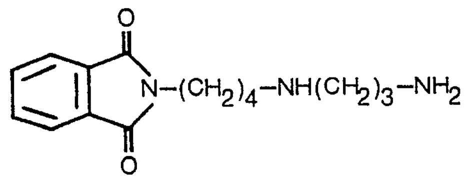

III

More specifically either with a protective group introducing agent to form a compound of the formula II

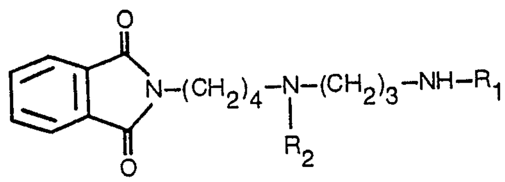

II

wherein $R_1$ and $R_2$ are identical, or first with a $R_2$-introducing agent in the presence of a Crown ether to form a compound of the formula IV

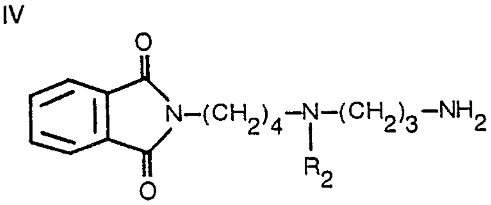

IV

and after removing the Crown ether, reacting the compound obtained with an $R_1$-introducing agent to form a compound of the formula II wherein $R_1$ and $R_2$ are different.

The intermediates of the formula II can be used in a process for preparing 1,5-diprotected-1,5,10-triazadecanes of the formula I

$$H_2N{-}(CH_2)_4{-}\underset{\displaystyle R_2}{\underset{|}{N}}{-}(CH_2)_3{-}NH{-}R_1 \qquad \mathrm{I}$$

wherein $R_1$ and $R_2$ are identical or different and represent an amino-protecting group other than a phthalyl group, which process comprises reacting 10-phthalyl-1,5-diprotected-1,5,10-triazadecanes of the general formula II

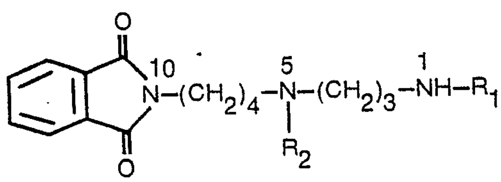

II

wherein $R_1$ and $R_2$ are as defined above with hydrazine or a hydrazine derivative to eliminate the phthalyl group.

1,5-diprotected-1,5,10-triazadecanes of the general formula (I) are very useful compounds for synthesizing 10-substituted 1,5,10-triazadecanes ($N^3$-substituted spermidines). Spergualin, for example, is an antitumor substance isolated from culture broth of a microorganism (T., Takeuchi et al., J. Antibiotics, 34, 1619)). This substance, having the following structural formula (H, Umezawa et al. J. Antibiotics, 34, 1622 (1981)),

$$H_2N\underset{\displaystyle NH}{\underset{\|}{C}}NH(CH_2)_4\,\underset{\displaystyle OH}{\underset{|}{C}}HCH_2CONH\,\underset{\displaystyle OH}{\underset{|}{C}}HCONH(CH_2)_4NH(CH_2)_3NH_2$$

was synthesized from 1,5-di-tert-butoxycarbonyl-1,5,10-triazadecane (S. Kondo et al., J. Antibiotics, 34, 1625 (1981)). The hitherto known compound of the general formula (I) has been 1,5-di-tert-butoxycarbonyl-1,5,10-triazadecane disclosed in the above reference. This compound was synthesized by a 6-step method in accordance with the following reaction scheme:

$$\underset{(A)}{HO(CH_2)_3NH_2} \xrightarrow{\text{Boc-introducing agent}} \underset{(B)}{HO(CH_2)_3NH\text{-}Boc} \xrightarrow{TosCl}$$

$$\underset{(C)}{Tos\text{-}O(CH_2)_3NH_2\text{-}Box} \xrightarrow{LiBr} \underset{(D)}{Br(CH_2)_3NH\text{-}Boc}$$

$$\xrightarrow{Z\text{—}NH(CH_2)_4NH_2} \underset{(E)}{Z\text{—}NH(CH_2)_4NH(CH_2)_3NH\text{-}Boc}$$

$$\xrightarrow{\text{Boc-introducing agent}} \underset{(F)}{Z\text{—}NH(CH_2)_4\underset{Boc}{N}(CH_2)_3NH\text{-}Boc} \xrightarrow{H_2/Pd}$$

$$\underset{(G)}{H_2N(CH_2)_4\underset{\substack{|\\Boc}}{N}(CH_2)_3NH\text{-}Boc}$$

In detail, 3-aminopropanol (A) is tert-butoxycarbonyl(Boc)ated with tert-butyl-4,6-dimethylpyrimidin-2-yl-thiolcarbonate to form thė compound (B). p-Toluenesulfonyl (Tos) chloride is caused to act on (B) to form (C). (C) is reacted with benzyloxycarbonyl(Z) aminobutylamine to form (E). (E) is tert-butoxycarbonyl(Boc)ated with tert-butyl-4,6-dimethylpyrimidine-2-ylthiolcarbonate to form (F). (F) is catalytically reduced with 5% palladium-barium carbonate to form 1,5-di-tert-butoxycarbonyl-1,5,10-triazadecane (G).

Now, it has been found that the compounds of the formula I can be synthesized easily from triazadecane (spermidine) and that this synthesis needs less steps than the known process. The present invention will be described in more detail below.

In compound of the general formula (II), the starting materials of this invention, $R_1$ and $R_2$ are each an amino-protecting group other than a phthalyl group and may be the same or different. Their examples include an aryl-$C_1$—$C_3$-alkoxycarbonyl group and an $C_1$—$C_6$-alkoxycarbonyl group. The phenyl group or alkyl group in these groups may have a substitutent. Examples of the substituents for the phenyl group are halogen atoms, the nitro group, methyl or methoxy. Examples of the substituent for the $C_1$—$C_6$-alkoxy group are halogen atoms. Preferred examples of the above-mentioned protective group are a benzyloxycarbonyl group or a benzyloxycarbonyl group having a substituent, such as a $C_1$—$C_3$ alkoxy group, a halogen atom or a nitro group, on the phenyl group (e.g., a p-methoxybenzyloxycarbonyl group, a p-chlorobenzyloxycarbonyl group, a p-bromobenzyloxycarbonyl group, or a p-nitrobenzyloxycarbonyl group); and a tert-alkoxycarbonyl group, such as a tert-butoxycarbonyl group or a tert-pentoxycarbonyl group.

Typical examples of the compounds expressed by the general formula (I) are listed below.

10-Phthalyl-1,5-dibenzyloxycarbonyl-1,5,10-triazadecane
10-Phthalyl-1,5-di-p-methoxybenzyloxycarbonyl-1,5,10-triazadecane
10-Phthalyl-1,5-di-p-chlorobenzyloxycarbonyl-1,5,10-triazadecane
10-Phthalyl-1,5-di-p-bromobenzyloxycarbonyl-1,5,10-triazadecane
10-Phthalyl-1,5-di-p-nitrobenzyloxycarbonyl-1,5,10-triazadecane
10-Phthalyl-1,5-di-tert-butoxycarbonyl-1,5,10-triazadecane
10-Phthalyl-1,5-di-tert-pentoxycarbonyl-1,5,10-triazadecane
10-Phthalyl-1-tert-butoxycarbonyl-5-benzyloxycarbonyl-1,5,10-triazadecane
10-Phthalyl-1-benzyloxycarbonyl-5-tert-butoxycarbonyl-1,5,10-triazadecane

Elimination of the phthalyl group from these compounds may be carried out by any of known methods such as disclosed in the following references: Shiroh Akabori, Takeo Kaneko and Kohzoh Narita: Tanpakushitsu Kagaku (Protein Chemistry) 1, Amino acids Peptides, Kyohritsu Shuppan, 1969; Nobuo

Izumiya, Tetsuo Katoh, Motoyoshi Ohno and Akihiko Aoyagi: Peptide Gosei (Peptides Synthesis), Maruzen, 1975; E. Schröder and K. Lübke: The Peptides, Academic Press, New York, 1965; E. Wüsch: Methoden der Organischen Chemie (Houben-Weyl), Synthese von Peptiden, George Thieme Verlag Stuttgart, 1974; and M. Bodanszky and M. A. Ondetti: Peptide Synthesis, Interscience Publishers, New York, 1976. Specifically, the phthalyl group can be eliminated by reacting compounds of the general formula (II) with hydrazines or their derivatives in ordinary organic solvents or hydrous organic solvents. The organic solvents or hydrous organic solvents used for the above purposes are not restricted to specific types. Their examples include lower alcohols with 1 to 4 carbon atoms, such as methanol, ethanol, propanol, isopropanol and n-butanol; ethers such as tetrahydrofuran and dioxane; and mixtures of water and aromatic organic solvents such as benzene, toluene and xylene.

Examples of the hydrazines are hydrazine, hydrazine hydrate, organic acid salts or inorganic acid salts of hydrazine, lower alkyl hydrazines such as methylhydrazine, and phenylhydrazine.

A preferred method of eliminating the phthalyl group is to subject compounds of the general formula (II) to the action of hydrazines, such as hydrazine, hydrazine hydrate or hydrazine acetate, in lower alcohols such as methanol or ethanol. The amount of the hydrazine used is 1 equivalent or more per equivalent of the phthalyl compound, but preferably, it should be about 1 to 5 equivalents.

The reaction temperature is usually above room temperature, preferably, from 50°C to the boiling point of the solvent. The isolation of the 1,5-diprotected-1,5,10-triazadecane of the general formula (I) from the reaction mixture can be performed in a known manner. In case the phthalyl group has been eliminated using hydrazine in ethanol, for example, the isolation can be effected as follows: The reaction mixture is concentrated to dryness under reduced pressure. Water is added to the residue, and the mixture is adjusted to a pH of 2 or 3 by addition of hydrochloric acid. The insolubles are removed by filtration, and the filtrate is adjusted to a pH of 10 by addition of an aqueous solution of sodium hydroxide. This liquid extracted with ethyl acetate, and the ethyl acetate layer is washed with water. The washed layer is dried over anhydrous sodium sulfate, and the desiccant is removed by filtration. The filtrate is concentrated under reduced pressure to obtain the compound of the general formula (I).

If the reaction mixture does not adversely affect a next reaction, it can be used as such in the next reaction. Typical examples of 1,5-diprotected-1,5,10-triazadecanes so obtained are given below.

1,5-Dibenzyloxycarbonyl-1,5,10-triazadecane
1,5-Di-p-methoxybenzyloxycarbonyl-1,5,10-triazadecane
1,5-Di-p-chlorobenzyloxycarbonyl-1,5,10-triazadecane
1,5-Di-p-bromobenzyloxycarbonyl-1,5,10-triazadecane
1,5-Di-p-nitrobenzyloxycarbonyl-1,5,10-triazadecane
1,5-Di-tert-butoxycarbonyl-1,5,10-triazadecane
1-Tert-butoxycarbonyl-5-benzyloxycarbonyl-1,5,10-triazadecane
1-Benzyloxycarbonyl-5-tert-butoxycarbonyl-1,5,10-triazadecane

10-Phthalyl-1,5-diprotected-1,5-10-triazadecanes of the general formula (II), the starting compounds of this invention, are novel compounds that have not been disclosed in the literature. The compound of the general formula (II), in which the substituents $R_1$ and $R_2$ are the same, can be synthesized from known 1-(4-aminobutyl)hexahydropyrimidine (1) — obtained by reacting formalin with spermidine — by three steps in accordance with the following reaction scheme:

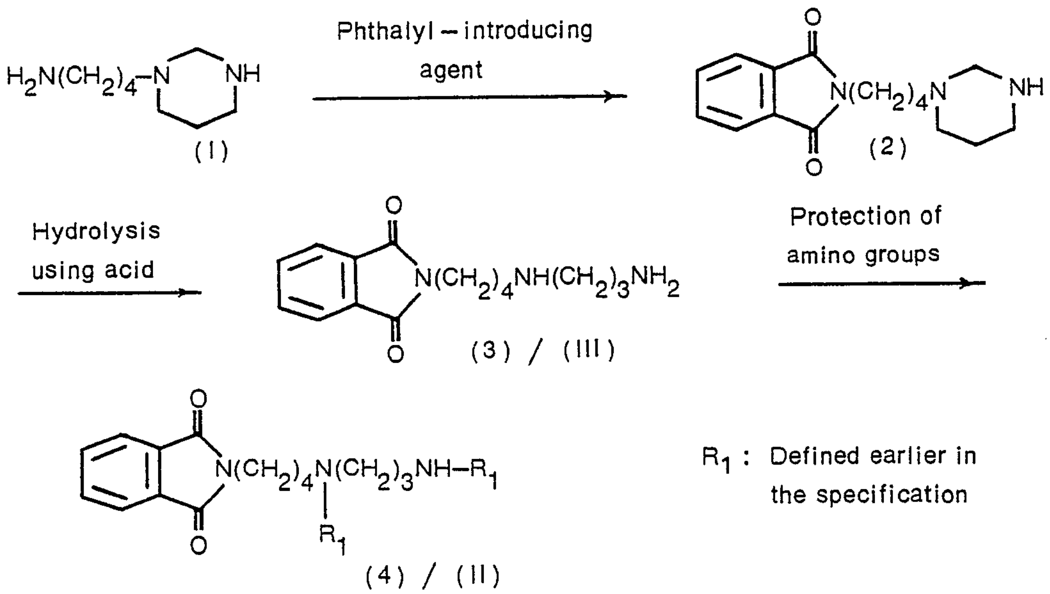


(1) (2) (3) / (III) (4) / (II)

$R_1$ : Defined earlier in the specification

$R_1$ and $R_2$: Defined earlier in the specification.

In detail, N-ethoxycarbonylphthalide, for example, is used to introduce a phthalyl group into (1), thereby forming 1(4-phthaliminobutyl)hexahydropyrimidine (2). (2) is hydrolyzed in the presence of hydrochloric acid to form 10-phthalyl-1,5,10-triazadecane (3). Then, a protective agent for the amino groups is caused to act on (3), to form the compound (4) expressed by the formula (II) in which $R_1$ and $R_2$ are the same.

Compounds of the general formula (II) in which the substituents $R_1$ and $R_2$ are different can be synthesized from 10-phthalyl-1,5,10-triazadecane (3) by 2 steps in accordance with the following reaction scheme:

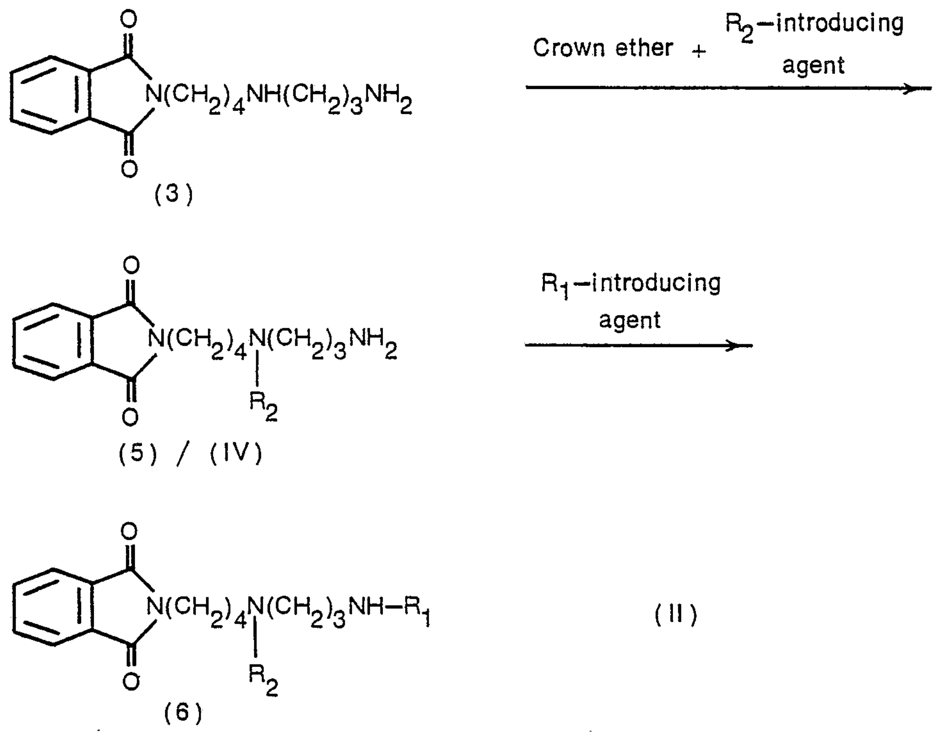


(3)

(5) / (IV)

(II)

(6)

$R_1$ and $R_2$: Defined earlier in the specification.

Detailedly speaking, Crown ether (e.g. 18-Crown-6) is added to 10-Phthalyl-1,5,10-triazadecane for the reversible protection of the primary amino group, and an agent for introducing $R_2$ is reacted with the secondary amino group to form 1,5-diprotected-triazadecane (5). Then, the Crown ether is removed, followed by reacting an $R_1$-introducing agent with the deprotected amino group to form the compound (6) of the general formula (II) in which $R_1$ and $R_2$ are different.

The present invention will be described in more detail with reference to working examples. In these examples, Rf values on thin-layer chromatography (TLC) were obtained by spotting a sample solution on a plate of silica gel (Silical Gel 60 $F_{254}$, plate thickness: 0.25 mm, a product of Merck) or a plate of alumina (Aluminium oxide 60 $F_{254}$, plate thickness: 0.25 mm, a product of Merck), developing the plate for a distance of about 8 cm with the developer described in the example, and dividing the distance from the origin of the spotting to the center of the resulting spot of the desired substance by; the distance from the origin of the spotting to the front end of the developer region on the plate. The spot of the desired substance was detected using ninhydrin and UV absorption spectrum (2537 A).

## Example 1

(a) 27.9 grams (80.0 mmols) of 10-phthalyl-1,5,10-triazadecane dihydrochloride was suspended in 300 ml of chloroform. 43.9 g (160 mmols) of benzyl S—4,6-dimethylpyrimidine-2-ylthiocarbonate and 17.8 g (176 mmols) of triethylamine were added to the suspension. The mixture was reacted at room temperature for 6 hours with stirring. The reaction mixture was washed sequentially with 1N hydrochloric acid and an aqueous solution of sodium chloride. The washed liquid was dried over anhydrous magnesium sulfate. The residue was concentrated under reduced pressure to obtain 43.1 g (yield: quantitative) of 10-phthalyl-1,5-dibenzyloxycarbonyl-1,5,10-triazadecane as a pale yellow oil.

NMR ($CDCl_3$)

δ= 1,3—2,1 (b, 6H), 1.9—3.9 (b, 8H), 5.1 ((b, 2H), 5.1 (S. 5H), 7.30, 73.33 (S, S10H), 7.73 (m, 4H)

IR (Neat)

$\nu(cm^{-1})$= 3350, 2950, 1770, 1720, 1525, 1395, 1245, 1150, 1040, 715, 690

TLC (n-propanol:water: 29% ammonium hydroxide= 10:1:0.15 v/v) Rf= 0.4 (silica gel plate)

b) 31.3 g (57.6 mmols) of 10-phthalyl-1,5-dibenzyloxycarbonyl-1,5,10-triazadecane was dissolved in 600 ml of ethanol. 18.2 g (291 mmols) of 80% hydrazine hydrate was added to the solution. The mixture was

heated overnight under reflux. Crystals that formed as precipitates were removed by filtration. The filtrate was concentrated under reduced pressure. The residue was dissolved in 300 ml of ethyl acetate. The solution was extracted with dilute hydrochloric acid in an attempt to obtain the desired product. The aqueous layer was washed with ethyl acetate. Then, sodium carbonate was added to adjust the liquid to a pH of 10. The oil that resulted was extracted with 500 ml of ethyl acetate. The extract was washed with a saturated aqueous solution of sodium chloride. The washed liquid was concentrated under reduced pressure to give 20.1 g (yield: 84.8%) of 1,5-dibenzyloxycarbonyl-1,5,10-triazadecane as a pale yellow oil.

NMR ($CDCl_3$)
δ= 1.0—2.3 (b, 8H), 2.3—2.9 (b, 8H), 2.9—3.5 ((b, 6H), 5.05 (S. 2H), 5.07 (S, S2H), 5.1—6.1 7.30 (S, 10H)
IR (Neat)
ν($cm^{-1}$)= 3340, 3335, 2940, 1695, 1530, 1475, 1450, 1420, 1245
TLC (n-propanol:water: 29% ammonium hydroxide= 10:1:0.15 v/v
Rf= 0.1 (silica gel plate), 0.4 (alumina plate)

Example 2

(a) 2.85 g (8.18 mmols) of 10-phthalyl-1,5,10-triazadecane dihydrochloride was suspended in 40 ml of chloroform. 5.48 g (18.0 mmols) of p-methoxybenzyl S—4,6-dimethylpyrimidin-2-yl-thiocarbonate and 1.82 g (18.0 mmols) of triethylamin were added to the suspension. The mixture was reacted for 6 hours at room temperature with stirring.

The reaction mixture was washed sequentially with 0.5N hydrochloric acid and an aqueous solution of sodium chloride. The washed liquid was dried over anhydrous sodium sulfate. The residue was concentrated under reduced pressure to give 4.70 g (yield: 95.1%) fo 10-phthalyl-1,5-di-p-methoxybenzyloxycarbonyl-1,510-triazadecane as a pale yellow oil.

NMR ($CDCl_3$)
δ= 1.3—2.0 (b, 6H), 2.8—3.5 (b, 6H), 3.5—3.9 (b, 2H), 3.78 (S.6H), 5.00 (S, 4H), 5.0—6.0 (b, 1H), 6.6—7.0 (m, 4H), 7.1—7.5 (m, 4H), 7.5—8.0 (m, 4H)
IR (Neat)
ν($cm^{-1}$)= 3370, 2945, 1770, 1615, 1590, 1515, 1400, 1250, 1035, 825, 755, 727
TLC (n-propanol:water: 29% ammonium hydroxide= 10:1:0.15 v/v)
Rf= 0.8 (silica gel plate)

(b) 1.00 g (1.66 mmols) of 10-phthalyl-1,5-di-p-methoxybenzyloxycarbonyl-1,5,10-triazedecane was dissolved in 30 ml of ethanol. 0.530 g (8.47 mmols) of 80% hydrazine hydrate was added to the solution. The mixture was heated overnight under reflux. Crystals that formed as precipitates were removed by filtration. The filtrate was concentrated under reduced pressure. The residue was dissolved in 50 ml of ethyl acetate. The insoluble were removed by filtration. 50 ml of distilled water was added to the filtrate. With the mixture being stirred, phosphoric acid was added to adjust the pH to 5. The aqueous layer was separated, followed by adding to it sodium carbonate to adjust the pH to 10. The mixture was extracted with ethyl acetate. The ethyl acetate layer was washed with distilled water. The washed liquid was dried over anhydrous sodium sulfate. The residue was concentrated under reduced pressure to give 0.550 g (yield: 72.4%) of 1,5-di-p-methoxybenzyloxycarbonyl-1,5,10-triazadecane as a pale yellow oil.

NMR ($CDCl_3$)
δ= 1.0—2.1 (m, 8H), 2.5—2.8 (5, 2H, J=6Hz), 2.9—3.6 (m.6H), 3.8 (s, 6H), 5.03 (s, 4H), 5.2—5.8 (b, 1H), 6.6—7.0 (m, 4H), 7.1—7.5 (m, 4H)
IR (Neat)
ν($cm^{-1}$)= 3350, 2940, 1690, 1615, 1590, 1515, 1305, 1245, 1175, 1030, 825
TLC (n-propanol:water: 29% ammonium hydroxide= 10:1:0.15 v/v) Rf= 0.7 (alumina plate)

Example 3

(a) 502 mg (1.44 mmols) of 10-phthalyl-1,5,10-triazadecane dihydrochloride was dissolved in 15 ml of chloroform. 762 mg (3.15 mmols) of tert-butyl S-4,6-dimethylpyrimidine-2-ylthiocarbonate and 321 mg (3.17 mmols) of triethylamin were added to the solution. The mixture was reacted overnight at room temperature with stirring. The reaction mixture was washed sequentially with 5% phosphoric acid and an aqueous solution of sodium chloride. The washed liquid was dried over anhydrous sodium sulfate. The residue was concentrated under reduced pressure to obtain 602 mg (yield: 88.0%) of 10-phthalyl-1,5-di-tert-butyloxycarbonyl-1,5,10-triazadecane which was dissolved in 30 ml of ethanol. 0.45 g (7.19 mmols) of 80% hydrazine hydrate was added to the solution. The mixture was heated overnight under reflux. Crystals that formed as precipitates were removed by filtration. The filtrate was concentrated under reduced pressure. 30 ml of ethyl acetate and 30 ml of distilled water were added to the residue. Phosphoric acid was added to the mixture to adjust it to a pH of 5. The aqueous layer was separated and washed with ethyl acetate. Sodium bicarbonate was added to the washed liquid to adjust it to a pH of 10. The mixture was

extracted with 100 ml of ethyl acetate. The ethyl acetate layer was washed with a saturated aqueous solution of sodium chloride.

The washed liquid was dried over anhydrous sodium sulfate. The residue was concentrated under reduced pressure to obtain 0.25 g (yield: 62.6%) of 1,5-ditert-butoxycarbonyl-1,5,10-triazadecane as a pale yellow oil.

NMR ($CDCl_3$)
δ= 1.0—2.0 (b, 8H), 1.48 (s, 18H), 2.3—2.9 (b, 2H), 2.9—3.5 (m, 6H), 4.7—5.6 (b, 1H)
IR (KBr)
ν($cm^{-1}$)= 3355, 2975, 2925, 2865, 1690, 1415, 1360, 1245, 1160
TLC (n-propanol:water:20% ammonium hydroxide= 10:1:0.15 v/v) Rf= 0.5 (alumina plate).

Example 4

(a) 0.350 g (1.0 mmols) of 10-phthalyl-1,5,10-triazadecane dihydrochloride was suspended in 2 ml of chloroform. 0.530 g (2.0 mmols) of 18-Crown-6 was added to the suspension. With the mixture being cooled with ice water, 0.171 g (1 mmol) of benzyloxycarbonyl chloride and 0.303 g (3 mmols) of triethylamine were added. The reaction system was returned to room temperature, and reacted for 3 hours with stirring. The reaction mixture was diluted with 35 ml of chloroform. The dilution was washed sequentially with water and a saturated aqueous solution of sodium bicarbonate, water, 1N hydrochloric acid and water. The chloroform layer was dried over anhydrous sodium sulfate. The residue was concentrated under reduced pressure to give 0.730 g of a transparent oil.

0.700 g of the resulting oil was dissolved in 7.0 ml of chloroform. 0.745 g (10 mmols) of potassium chloride was added to the solution. With the mixture being cooled with ice water, 0.240 g (1 mmol) of tertbutyl-S-4,6-dimethylpyrimidin-2-yl-thiocarbonate and 0.101 g (1 mmol) of triethylamine were added. The reaction systems was brought back to room temperature, and reacted overnight at this temperature with stirring.

The reaction mixture was diluted with 30 ml of chloroform. The dilution was washed sequentially with water, a 10% aqueous solution of phosphoric acid, a saturated aqueous solution of sodium bicarbonate, and water. The washed liquid was dried over anhydrous magnesium sulfate. The residue was concentrated under reduced pressure to yield 0.560 g of a yellow oil. The resulting oil was chromatographed on a 40 g column of silica gel (Wako Gel C—200). The column was developed with benzene-ethyl acetate (5:1 v/v). 0.210 g of 1-tert-butoxycarbonyl-5-benzyloxycarbonyl-10-phthalyltriazadecane was obtained as an oil in a yield of 43.0%.

NMR ($CDCl_3$)
δ= 1.47 (s, 9H), 1.1—2.0 (m, 6H), 2.9—3.5 (m, 6H), 3.67 (b, 2H), 4.4—5.5 (b, 1H), 5.12 (s, 2H), 7.32 (s, 5H), 7.75 (m, 4H)
IR (Neat)
ν($cm^{-1}$)= 3370, 2940, 1770, 1715, 1550, 1475, 1420, 1390, 1365, 1250, 1165, 1035, 720, 680
TLC (benzene:ethyl acetate = 5:1 v/v)
Rf= 0.2 (silica gel plate)

(b) 0.080 g (0.15 mmols) of 10-phthalyl-1-tert-butoxycarbonyl-5-benzyloxycarbonyl-1,5,10-triazadecane was dissolved in 2 ml of ethanol. 0.063 g (1.00 mmols) of 80% hydrazine hydrate was added to the solution. The mixture was heated overnight under reflux. Crystals that formed as precipitates were removed by filtration. The filtrate was concentrated under reduced pressure. The residue was dissolved in 30 ml of ethyl acetate. The solution was extracted with 30 ml of a 5% aqueous solution of phosphoric acid. The aqueous layer was washed with ethyl acetate. The washed liquid was adjusted to a pH of more than 10 by addition of sodium carbonate. The mixture was extracted with 50 ml of ethyl acetate. The ethyl acetate layer was washed with a saturated aqueous solution of sodium chloride, and dried over anhydrous sodium sulfate.

The residue was concentrated under reduced pressure to obtain 0.030 g of 1-tert-butoxycarbonyl-5-benzyloxycarbonyl-1,5,10-triazadecane as an oil.
Yield: 54.9%.

NMR ($CDCl_3$)
δ= 0.3—2.4 (b, 8H), 1.43 (s, 9H), 2.4—2.9 (b, 2H) 2.9—3.5 (m, 6H), 4.5—5.6 (b, 1H), 5.10 (s, 2H), 7.33 (s, 5H)
IR (Neat)
ν($cm^{-1}$)= 3350, 2930, 1700, 1520, 1475, 1420, 1360, 1250, 1165
TLC (n-propanol:water: 29% ammoniumhydroxide= 10:1:0.15 v/v RF= 0.6 (alumina plate)

Referential Example

Synthesis of 10-phthalyl-1,5,10-triazadecane dihydrochloride

(a) 290 g (2.00 mols) of spermidine was dissolved in 500 ml of water. With the solution being cooled with ice water, 162 g (2.00 mols) of 37% formalin was added. The reaction system was reacted overnight at room temperature with stirring. The reaction mixture was concentrated under reduced pressure. The

residue was distilled in vacuo. The distillates at boiling points of 90—92°C/2 mm Hg were collected. 63 g of 1-(4-aminobutyl)hexahydropyrimidine was obtained as translucent needles. Yield: 83.8%
Mp: 39—42°C

NMR ($CDCl_3$)
δ= 1.1—1.9 (m, 9H), 2.0—2.4 (m, 2H), 2.4—3.0 (m, 6H), 3.34 (s, 2H)
IR (KBr)
ν($cm^{-1}$)= 3315, 3265, 3215, 2940, 1620, 1465, 1375, 1300, 1235, 1205, 1155, 1100, 1000

(b) 55.0 g (350 mmols) of 1-(4-aminobutyl)hexahydropyrimidine was dissolved in 580 ml of dimethyl sulfoxide. With the solution being cooled, 42.0 g (700 mmols) of glacial acetic acid was added. Then, 92.0 g (420 mmols) of N-ethoxycarbonylphthalimide was added to the mixture. The reaction system was reacted overnight at room temperature with stirring. The reaction mixture was concentrated under reduced pressure by means of vacuum pump. The residue was dissolved in 200 ml of distilled water. The solution was adjusted to a pH of 1.0 by addition of concentrated hydrochloric acid. Then, the solution was concentrated under reduced pressure. The residue was recrystallized from ethanol. 46.9 g of 10-phthalyl-1,5,10-triazadecane dihydrochloride was obtained as pale yellow crystals.
Yield: 38.5%
Mp: 244—246°C

NMR ($D_2O$)
δ= 1.5—2.0 (b, 4H), 2.0—2.5 (m, H), 2.9—3.5 (b, 6H), 3.5—3.9 (b, 2H), 7.77 (s, 4H)
IR (KBr)
ν($cm^{-1}$)= 3430, 2930, 1775, 1710, 1465, 1435, 1400, 1380, 1050.

## Claims

1. A compound of the general formula II

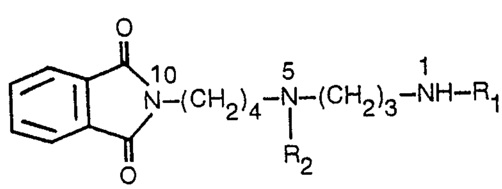

II

wherein $R_1$ and $R_2$ are identical or different and represent each an amino-protecting group other than the phthalyl group.

2. The compound as claimed in claim 1 wherein $R_1$ and $R_2$ represent aryl-$C_1$—$C_3$-alkoxycarbonyl wherein aryl represents a phenyl group which is unsubstituted or substituted with halogen, nitro, $C_1$—$C_3$-alkyl or -alkoxy, or $R_1$ and $R_2$ represent $C_1$—$C_6$-alkoxy carbonyl.

3. A process for preparing a compound of the formula II as claimed in claim 1 which comprises reacting a compound of the formula III

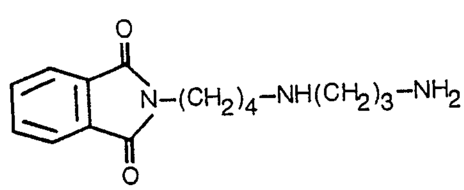

III

either with a protective group introducing agent to form a compound of the formula II

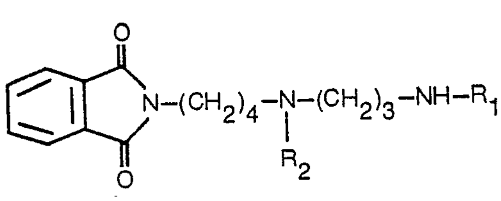

II

wherein $R_1$ and $R_2$ are identical, or first with a $R_2$-introducing agent in the presence of a Crown ether to form a compound of the formula IV

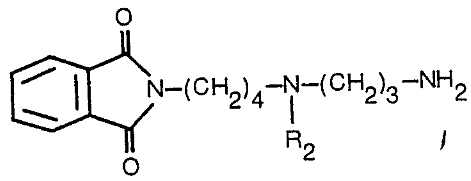

IV

and, after removing the Crown ether, reacting the compound obtained with an $R_1$ introducing agent to form a compound of the formula II wherein $R_1$ and $R_2$ are different.

4. Use of a compound of the formula II as defined in claim 1 for the preparation of a compound of the formula

$$H_2N—(CH_2)_4—N—(CH_2)_3—NH—R_1$$
$$R_2$$

wherein $R_1$ and $R_2$ are as defined in claim 1 by removing the phthalyl group in a conventional manner.

**Patentansprüche**

1. Verbindung der allgemeinen Formel II

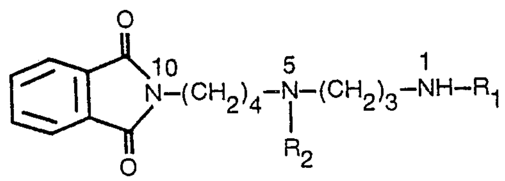

II

in der $R_1$ und $R_2$ identisch oder vershieden sind und jeweils eine von der Phthaloylgruppe verschiedene Amino-Schutzgruppe darstellen.

2. Verbindung gemäss Anspruch 1, in der $R_1$ und $R_2$ einen Aryl-($C_1$—$C_3$)alkoxycarbonylrest darstellen, in welchem der Arylrest eine unsubstituierte oder mit Substituenten wie Halogenatom, Nitrogruppe, ($C_1$—$C_3$) Alkyl- oder -Alkoxyrest versehene Phenylgruppe ist, oder in der $R_1$ und $R_2$ einen ($C_1$—$C_6$)-Alkoxy-carbonylrest darstellen.

3. Verfahren zur Herstellung einer Verbindung der Formel II gemäss Anspruch 1, dadurch gekennzeichnet, dass man eine Verbindung der Formel III

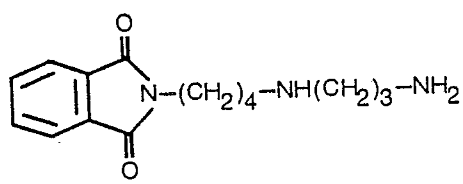

III

entweder durch Reaktion mit einem Mittel zur Einführung einer Schutzgruppe in eine Verbindung der Formel II überführt, in welcher $R_1$ und $R_2$ identisch sind,

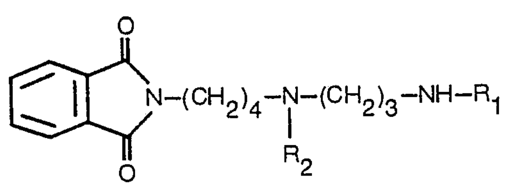

II

oder zuerst durch Reaktion mit einem Mittel zur Einführung des Restes $R_2$ in Gegenwart eines Kronenethers in eine Verbindung der Formel IV überführt

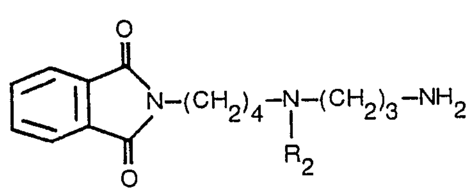

IV

und nach Entfernung des Kronenethers die erhaltene Verbindung durch Reaktion mit einem Mittel zur Einführung des Restes $R_1$ in eine Verbindung der Formel II überführt, in der $R_1$ und $R_2$ verschieden sind.

4. Verwendung einer Verbindung der Formel II gemäss Anspruch 1 zur Herstellung einer Verbindung der Formel

$$H_2N{-}(CH_2)_4{-}\underset{\displaystyle R_2}{\underset{|}{N}}{-}(CH_2)_3{-}NH{-}R_1$$

in der $R_1$ und $R_2$ die in Anspruch 1 gegebene Bedeutung haben, durch Entfernung der Phthaloylgruppe in bekannter Weise.

**Revendications**

1. Composé de formule générale II:

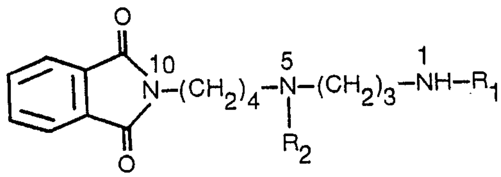

II

dans lequel les groupes $R_1$ et $R_2$ sont identiques ou différents, et ils représentent chacun un groupe amino-protecteur autre que le groupe phtalyle.

2. Composé suivant la revendication 1, dans lequel les groupes $R_1$ et $R_2$ représentent un groupe aryl alkoxy ($C_1$—$C_3$) carbonyle, dans lequel le groupe aryle représente un groupe phényle non substitué ou substitué avec un atome d'halogène, un groupe nitro, alkyl ($C_1$—$C_3$) ou alkoxy ($C_1$—$C_3$), ou $R_1$ et $R_2$ représentent un groupe alkoxy ($C_1$—$C_6$) carbonyle.

3. Procédé de préparation d'un composé de formule II suivant la revendication 1, dans lequel on fait réagir un composé de formule III:

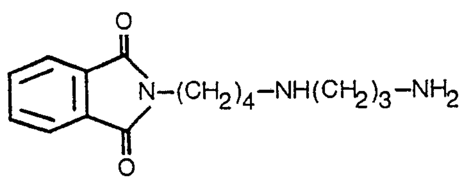

III

soit avec un agent d'introduction d'un groupe protecteur pour former un composé de formule II

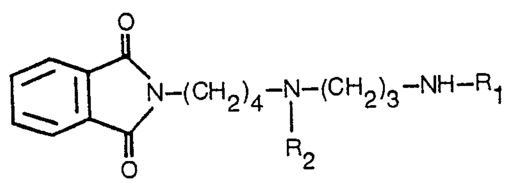

II

dans laquelle $R_1$ et $R_2$ sont identiques, ou d'abord avec un agent d'introduction d'un groupe $R_2$ en présence d'éther-couronne pour former un composé de formule IV:

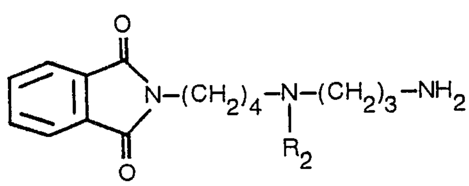

IV

et, après élimination de l'éther-couronne, on fait réagir le composé obtenu avec un agent d'introduction d'un groupe $R_1$ pour former un composé de formule II dans laquelle $R_1$ et $R_2$ sont différents.

4. Utilisation d'un composé de formule II tel que défini dans la revendication 1 pour la préparation d'un composé de formule:

$$H_2N{-}(CH_2)_4{-}\underset{\displaystyle R_2}{\underset{|}{N}}{-}(CH_2)_3{-}NH{-}R_1$$

dans laquelle $R_1$ et $R_2$ sont tels que définis dans la revendication 1 en éliminant le groupe phtalyle de façon classique.